# EUROPÄISCHE PATENTSCHRIFT

(11) **EP 3 123 136 B1**
(45) Veröffentlichungstag und Bekanntmachung des Hinweises auf die Patenterteilung: **13.02.2019**
(21) Anmeldenummer: 15747979.1
(22) Anmeldetag: 08.07.2015
(51) Int. Cl.: G01M 3/26, G01N 15/08, G01N 17/00, G01M 3/18, G01M 3/22, G01M 3/32, C12Q 1/04

(54) **VORRICHTUNG UND VERFAHREN ZUR DURCHFÜHRUNG EINES INTEGRITÄTSTESTS**
DEVICE AND METHOD FOR CARRYING OUT AN INTEGRITIY TEST
DISPOSITIF ET PROCÉDÉ DE TEST D'INTÉGRITÉ

(30) Priorität: 17.07.2014 DE 102014010559; 09.01.2015 DE 102015000314
(43) Veröffentlichungstag der Anmeldung: 01.02.2017
(73) Patentinhaber: Sartorius Stedim Biotech GmbH, 37079 Göttingen (DE)
(72) Erfinder: DAHLBERG, Martin, 37120 Bovenden (DE); GAY, Isabelle, F-13124 Peypin (FR); MENIER, Marie Christine, 13600 La Ciotat (FR); MONTENAY, Nelly, 13790 Rousset (FR)
(74) Vertreter: Müller-Boré & Partner Patentanwälte PartG mbB
(86) Internationale Anmeldenummer: PCT/EP2015/001390
(87) Internationale Veröffentlichungsnummer: WO 2016/008574

(56) Entgegenhaltungen:
- WO-A1-2008/030712
- DE-A1-102004 016 592
- FR-A1- 2 960 972
- US-A- 4 789 434
- US-A1- 2009 320 564
- US-A1- 2014 060 159

## Beschreibung

Die Erfindung betrifft eine Vorrichtung und ein Verfahren zur Durchführung eines Integritätstests an einer Probe von Einwegkomponenten sowie die Verwendung einer solchen Vorrichtung.

In der biopharmazeutischen Industrie erfolgt ein Wandel von Anlagen aus Edelstahlkomponenten hin zu Einwegkomponenten. Da Einwegkomponenten aus Kunststoff bei Herstellung, Transport und Handhabung einfacher beschädigt werden können als Anlagen aus Edelstahlkomponenten, wird von Kunden und Regulationsbehörden häufig ein Integritätstest der Einwegkomponenten gefordert. Ein Integritätstest kann dabei ein Lecktest sein, bei dem die Einwegkomponente auf ein Leck hin getestet wird und bei der die Einwegkomponente nicht zerstört wird. Alternativ oder zusätzlich kann als Integritätstest ein Nachweis der Funktionalität der Einwegkomponente als Sterilbarriere durchgeführt werden, zum Beispiel ein Bakterienrückhaltetest (abgekürzt: BCT vom Englischen "bacteria challenge test") bei dem die Einwegkomponente zerstört wird.

Gängige Bakterienrückhaltetests mittels Immersion gemäß ISO15747 und mittels Aerosol sind in ihrer Durchführung häufig in der Größe beschränkt, zum Beispiel durch begrenzte räumliche Ausdehnung einer dazu verwendeten Aerosolkammer. Weitere Schwierigkeiten bei der Durchführung eines BCT liegen in der Handhabung von Proben der Einwegkomponenten bei der Durchführung des Integritätstests ohne Kreuzkontamination durch die Versuchsdurchführenden.

Aus DE 10 2010 018 881 A1 ist ein Reinigungsverfahren für eine Prüfvorrichtung bekannt, bei der ausgewählte interne Volumina mit einem Reinigungsfluid gereinigt werden und mit einem Spülfluid gespült werden. Dabei kann eine Rückhaltevorrichtung eine vorgeschaltete Entlüftung aufweisen.

Dokument FR 2 960 972 A1 betrifft eine Testzelle mit einer Kammer, an der eine Testmembran befestigt werden kann. Über einen Einlass kann ein Testfluid in die Kammer eingebracht werden. Die Kammer kann weiterhin über ein Ventil entlüftet werden. In einer Ausführungsform ist eine zweite Kammer an der Außenseite der Testmembran angeordnet.

Dokument US 2009/0320564 A1 betrifft eine Vorrichtung zum Messen einer Gasdurchlässigkeit durch einen Film. Dazu wird der Film zwischen einer Basis und einem Deckel eingespannt. In dieser Position unterteilt der Film eine Kammer in zwei Räume, die jeweils einen Gaseinlass und einen Gasauslass aufweisen.

Dokument WO 2008/030712 A1 betrifft einen Apparat zum Testen einer Fluiddurchlässigkeit. Dabei wird ein Testfilm in eine Testzelle eingespannt, die dadurch in zwei Bereiche geteilt wird. Die zwei Bereiche sind mit einem Testgas oder einem Vakuum beaufschlagbar. Ein Massenspektrometer misst die Durchlässigkeit des Testfilms.

Dokument US 2014/0060159 A1 betrifft eine Vorrichtung zur Überprüfung der Durchlässigkeit von Dentin. Dabei wird eine Dentinprobe in einer Durchflusszelle zwischen einem Fuß und einem Deckel mittels Dichtungsringen eingeklemmt. Die Durchflusszelle kann so schräg gestellt werden, dass Luftbläschen über einen Kanal ausgelassen werden können.

Dokument US 4,789,434 betrifft eine Korrosionsmessvorrichtung zur Messung biologisch induzierter Korrosion mittels galvanisch gekoppelter Elektroden.

Aus der DE 101 16 335 C1 ist ein Verfahren zur Durchführung eines Integritätstests von Filterelementen bekannt, wobei ein konvektiver Anteil eines Gasvolumenstroms durch die Filterelemente aus einem Verhältnis von Gasvolumenströmen und einem Verhältnis von Löslichkeits- bzw. Diffusionskoeffizienten in einer Benetzungsflüssigkeit der Filterelemente zweier unterschiedlicher Testgase ermittelt wird. Hierbei kann eine Prüfvorrichtung verwendet werden, die Testgase aufnehmen kann.

Der Erfindung liegt die Aufgabe zugrunde, einen verbesserten und/oder vereinfachten Integritätstest an Proben von Einwegkomponenten zu ermöglichen.

Diese Aufgabe wird durch die Gegenstände der unabhängigen Ansprüche gelöst. Bevorzugte Ausführungsformen sind Gegenstände der abhängigen Ansprüche.

Ein erster Aspekt betrifft eine Vorrichtung zur Durchführung eines Integritätstests an einer Probe von Einwegkomponenten mit einer Mediumkammer mit einem nach außen begrenzten Innenraum zur Aufnahme eines Kulturmediums und mit einer Probenhalterung, in der die Probe so anordenbar ist, dass die Probe die Mediumkammer zumindest teilweise nach außen begrenzt. Dabei weist die Mediumkammer eine Entlüftungsleitung auf, die an einer der Probenhalterung abgewandten Seite der Mediumkammer angeordnet ist.

Als Probe einer Einwegkomponente kann hierbei ein ausgestanztes Stück Film oder Folie der Einwegkomponente mit oder ohne Schweißnaht dienen. In die Proben können weitere Komponenten wie zum Beispiel Fluidanschlüsse mit oder ohne zusätzlichen Schlauch eingeschweißt sein. Die Proben sind im Wesentlichen flächig ausgebildet und weisen eine Probeninnenfläche und eine gegenüberliegende Probenaußenfläche auf. Ist eine solche Probe in der Probenhalterung angeordnet, so ist die Probeninnenfläche dem Innenraum der Mediumkammer zugewandt, während die Probenaußenfläche nach außen gewandt ist und mit Bakterien und/oder Sporen beaufschlagbar ist. Insbesondere können die Proben scheibenförmig ausgebildet sein, z.B. mit einem Durchmesser von ca. 50 mm. Bei Verwendung von Proben solcher Größe kann die Vorrichtung selber eine begrenzte radiale Ausdehnung aufweisen, zum Beispiel von maximal Der Integritätstest kann insbesondere einen BCT enthalten, also einen Bakterienrückhaltetest, bei dem eine Kontamination der Mediumkammer durch die Probe hindurch untersucht wird. Der Integritätstest kann weiterhin mit einem Lecktest korreliert werden.

Zur Durchführung des Integritätstests wird die Mediumkammer mit einem Kulturmedium gefüllt, wie zum Beispiel mit TSB, einer tryptischen Sojaboullion. Die Mediumkammer ist dabei so leckfrei ausgebildet, dass sie ein zum Beispiel flüssiges Kulturmedium im Wesentlichen ohne relevante Verflüchtigung mehrere Tage lang halten kann. Der Innenraum der Mediumkammer kann dabei insbesondere mediendicht und/oder flüssigkeitsdicht ausgebildet sein.

Die Probe einer Einwegkomponente kann in der Probenhalterung so angeordnet werden, dass die Probe die Mediumkammer mediendicht verschließt. Dazu kann die Probenhalterung die Mediumkammer nach einer Seite hin zumindest teilweise begrenzen. Die Probe wird in der Probenhalterung angeordnet, zum Beispiel eingeklemmt. Die Probenhalterung kann Dichtelemente, zum Beispiel in Form von O-Ringen, aufweisen, die eine mediendichte Anordnung der Probe in der Probenhalterung ermöglichen. Die in der Probenhalterung angeordnete Probe stellt eine Grenzfläche der Mediumkammer bereit.

Die Entlüftungsleitung ist dazu ausgebildet und vorgesehen, die Mediumkammer von darin befindlichen Gasen zu entlüften, insbesondere von Luft. Die Entlüftungsleitung stellt eine Entlüftungsmöglichkeit an Seite der Mediumkammer bereit, die der Probenhalterung und somit der Probe abgewandt ist. Die Entlüftungsleitung kann z.B. an einer der Probenhalterung gegenüberliegenden Seitenwand der Medienkammer ausgebildet sein. So kann die Probenhalterung und die Probe in einer ersten Seite der Mediumkammer ausgebildet sein. Die Entlüftungsleitung setzt in dieser Ausführungsform an einer zweiten Seite der Mediumkammer an, die von der ersten Seite der Mediumkammer abgewandt ist und dieser ggf. gegenüber liegt.

In der der Probenhalterung abgewandten Seite der Mediumkammer ist dafür eine Öffnung ausgebildet, an der die Entlüftungsleitung ansetzt und über die der Innenraum der Mediumkammer entlüftet werden kann. In einer Ausführungsform ist die Entlüftungsleitung als ein verschließbares Ventil ausgebildet. In einer anderen Ausführungsform ist die Entlüftungsleitung als verschließbare rohr- oder schlauchförmige Leitung ausgebildet, die von einer Entlüftungsöffnung in der besagten Seite der Mediumkammer weg verlaufend angeordnet ist.

Durch die Anordnung der Entlüftungsleitung an der der Probenhalterung abgewandten Seite der Mediumkammer wird eine vollständige Befüllung der Mediumkammer mit dem Kulturmedium ermöglicht. Dazu kann die Mediumkammer so angeordnet sein, dass die Probenhalterung (im Bezugssystem der Erde) unten angeordnet ist, während die Entlüftungsleitung vertikal über der Probenhalterung angeordnet ist. Die Angaben "oben", "unten", "vertikal" und "horizontal" sind im Rahmen der Erfindung allgemein auf das Bezugssystem Erde bezogen, genauer auf die Gravitationskraft im Bezugssystem Erde. Über eine separate Medienleitung kann die Mediumkammer mit einem zum Beispiel flüssigen Kulturmedium gefüllt werden, das sich im Innenraum der Mediumkammer unmittelbar auf der Probe niederlässt. In der Mediumkammer befindliches Gas (wie z.B. Luft) ist leichter als das Kulturmedium und steigt deswegen im Innenraum der Mediumkammer nach oben, also in Richtung zu der Seite hin, an der die Entlüftungsleitung ansetzt. Bei geöffneter Entlüftungsleitung kann die Mediumkammer somit vollständig mit dem Kulturmedium gefüllt werden, während die im Innenraum der Mediumkammer befindliche Luft nach oben vollständig durch die Entlüftungsleitung entweichen kann.

Die Anordnung der Entlüftungsleitung gegenüber der Probe ermöglicht somit zum Einen eine unmittelbare Beaufschlagung der Probe, insbesondere der Probeninnenfläche, mit dem Kulturmedium, zum Anderen eine vollständige Befüllung der Mediumkammer mit dem Kulturmedium.

Dabei weist die Vorrichtung eine Ausrichtvorrichtung auf, mittels der die Mediumkammer in einer Befüllposition ausrichtbar ist, in der die Mediumkammer mit dem Kulturmedium befüllbar ist und in der die Probenhalterung vertikal unter der Entlüftungsleitung angeordnet ist. Die Ausrichtvorrichtung kann zum Beispiel als Schwenkvorrichtung ausgebildet sein, mit einer im Wesentlichen horizontal ausgerichteten Schwenkachse. Dabei ist die Mediumkammer um die Schwenkachse verschwenkbar, so dass die Probenhalterung am unteren Ende der Mediumkammer angeordnet ist, wobei die Entlüftungsleitung am oberen Ende der Mediumkammer angeordnet ist. Die Ausrichtvorrichtung ermöglicht insbesondere ein Anordnen des Angriffspunkts der Entlüftungsleitung an der Mediumkammer im Wesentlichen am obersten und/oder höchsten Punkt der Mediumkammer, so dass z.B. unter Atmosphärendruck vorliegende Gasblasen aus der Mediumkammer entweichen können, die ansonsten als Puffer zum Medium an einer Fehlstelle der Probe wirken könnten. Die Mediumkammer kann somit bei Befüllung mit einem Kulturme-dium vollständig gefüllt werden. Weiterhin kann die Ausrichtvorrichtung als eine Hal-terung der Mediumkammer ausgebildet sein.

Dabei ist die Mediumkammer mittels der Ausrichtvorrichtung zudem in einer Suspensionsbefüllposition ausrichtbar, in der die Probenhalterung vertikal oberhalb der Entlüftungsleitung angeordnet ist. In der Suspensionsbefüllposition stellt die Probe zumindest teilweise eine obere Seitenwand der Mediumkammer bereit. In der Suspensionsbefüllposition kann eine ggf. vorhandene Suspensionskammer besonders günstig mit einer Suspension gefüllt werden, wie weiter unten beschrieben.

Alternativ oder zusätzlich ist die Mediumkammer mittels der Ausrichtvorrichtung in einer Beaufschlagungsposition ausrichtbar. In der Beaufschlagungsposition kann die Probe besonders günstig mit einem Aerosol oder einer Suspension beaufschlagt werden, die Bakterien und/oder Sporen enthalt(en). In der Beaufschlagungsposition kann die Probenaußenfläche im Wesentlichen horizontal ausgerichtet sein und nach oben weisen. Alternativ ist in der Beaufschlagungsposition die Probenaußenfläche zur Horizontalen geneigt. Im letzteren Fall kann die Probe dabei mit ihrer Probenaußenfläche im Wesentlichen vertikal angeordnet sein oder mit einer Neigung zur Horizontalen, d.h. unter einem Winkel von 10° bis 90°, insbesondere von 40° und 90° zur Horizontalen.

Bei Beaufschlagung der Mediumkammer in der Suspensionsbefüllposition mit einem Aerosol kann das Aerosol auf der Probe ausfallen bzw. sich darauf niederschlagen. Bei Beaufschlagung mit einer Suspension in der Suspensionsbefüllposition wirkt die Gravitationskraft der Suspension direkt auf die an der Oberseite der Mediumkammer angeordnete Probe.

Die Suspensionsbefüllposition ist zum Befüllen der Suspensionskammer vorgesehen und unterscheidet sich durch die Ausrichtung der Vorrichtung von der Befüllposition, die zum Befüllen der Mediumkammer vorgesehen ist.

In der Befüllposition kann die Entlüftungsleitung der Mediumkammer am vertikal höchsten Punkt der Mediumkammer angeordnet sein und die Probenhalterung am vertikal tiefsten Punkt der Mediumkammer. In dieser Anordnung lässt sich die Mediumkammer besonders gut entlüften und die Probe wird besonders gut und flächig mit dem Kulturmedium bedeckt.

Gemäß einer Ausführungsform weist die Vorrichtung ein Verbindungselement zum Verbinden der Mediumkammer mit einer Suspensionskammer derart auf, dass die in der Probenhalterung angeordnete Probe eine Grenzfläche zwischen dem Innenraum der Mediumkammer und einem Suspensionskammerinnenraum der Suspensionskammer bereitstellt. Dabei kann die Mediumkammer von der Suspensionskammer lediglich über die in der Probenhalterung angeordnete Probe getrennt sein. Die Suspensionskammer kann mediendicht ausgebildet sein und zur Aufnahme einer Suspension dienen, die Bakterien und/oder Sporen enthält. Die Suspensionskammer kann über das Verbindungselement so an der Vorrichtung angeordnet werden, dass die Suspensionskammer an der Probenaußenfläche angeordnet ist, also der Seite der Probe, die von der Mediumkammer abgewandt ist. Die Probe kann als Grenzfläche zwischen der Mediumkammer und der Suspensionskammer angeordnet werden. Ist in der Suspensionskammer eine Bakterien- und/oder Sporensuspension angeordnet, so wird die Probe direkt von der Suspension beaufschlagt. Dadurch kann im Integritätstest überprüft werden, ob die Probe durchlässig für die in der Suspension befindlichen Bakterien und/oder Sporen ist. Ist die Probe durchlässig, so kann ein Wachstum im Kulturmedium beobachtet werden, dass in der Mediumkammer angeordnet ist. Das Verbindungselement kann zum Beispiel als Gewinde, Rastnase und/oder Einsteckelement ausgebildet sein. An der Suspensionskammer kann ein entsprechendes konträres Verbindungselement angeordnet sein. Das Verbindungselement kann insbesondere an der Probenhalterung ausgebildet sein und zum Verbinden der Suspensionskammer mit der Probenhalterung dienen. In diesem Fall kann die Mediumkammer lediglich über die Probenhalterung mit der Suspensionskammer verbunden werden. Die Verbindung zwischen der Suspensionskammer und der Mediumkammer kann lösbar ausgebildet sein. Dazu kann sowohl die Verbindung der Mediumkammer mit der Probenhalterung lösbar sein, als auch die Verbindung der Suspensionskammer mit der Probenhalterung. Ist die Suspensionskammer mit der Mediumkammer verbunden und eine Probe in die Probenhalterung eingebracht, so ist die Probe als Grenzfläche zwischen der Suspensionskammer und der Mediumkammer angeordnet.

In einer alternativen Ausführungsform kann die Mediumkammer und die Suspensionskammer als zusammenhängendes Bauteil ausgebildet sein, in das die Probenhalterung so einschiebbar ist, dass die Probe eine Grenze zwischen der Mediumkammer und der Suspensionskammer bildet.

In einer Weiterbildung der Ausführungsform weist die Suspensionskammer eine Suspensionskammerentlüftungsleitung auf, die an einer der Probenhalterung abgewandten Seite der Suspensionskammer angeordnet ist. Ähnlich wie die für das Befüllen der Mediumkammer mit dem Kulturmedium vorteilhaft positionierte Entlüftungsleitung der Mediumkammer, ist die Suspensionskammerentlüftungsleitung an der Seite der Suspensionskammer angeordnet, die von der Probenhalterung abgewandt ist und/oder ihr gegenüberliegt. Diese Anordnung der Suspensionskammerentlüftungsleitung ermöglicht ein vollständiges Befüllen der Suspensionskammer mit einer Suspension, wenn die Suspensionskammerentlüftungsleitung vertikal oberhalb der Probenhalterung angeordnet wird. Dabei kann die Suspensionskammerentlüftungsleitung zum Beispiel in der oben beschriebenen Suspensionsbefüllposition am vertikal höchsten Punkt der Suspensionskammer angeordnet werden und die Probenhalterung im Wesentlichen am vertikal tiefsten Punkt der Suspensionskammer.

Gemäß einer Ausführungsform ist in einer Seitenwand der Mediumkammer zumindest ein transparenter Bereich ausgebildet. Der Bereich ist lichtdurchlässig und dient zur Beobachtung des in der Mediumkammer angeordneten Kulturmediums. Über den transparenten Bereich kann ein Wachstum und somit eine Kontamination der Mediumkammer detektiert werden. In der Mediumkammer können insbesondere zwei gegenüberliegende transparente Bereiche ausgebildet sein, so dass eine Kontamination der Mediumkammer an die Mediumkammer durchdringendem Licht erkannt werden kann. Zur Erkennung einer Verfärbung und/oder Verdunklung des Kulturmediums kann eine Kontaminationssonde vorgesehen sein, die die Kontamination am transparenten Bereich automatisch überprüft.

Gemäß einer Ausführungsform ist die Probenhalterung in einer lösbaren Verbindung mit der Mediumkammer angeordnet und/oder gekoppelt. Ist die Probenhalterung von der Mediumkammer gelöst, kann zum Beispiel die Probe in die Probenhalterung eingebracht werden und/oder die Probe einem physikalischen Integritätstest wie einer Beaufschlagung mit Druck oder mit Spannung ausgesetzt werden. Die Probenhalterung ist dabei so ausgebildet, dass die Probe auch dann von der Probenhalterung sicher gehalten wird, wenn diese von der Mediumkammer und gegebenenfalls der Suspensionskammer getrennt ist.

Der physikalische Integritätstest an der Probe kann in einer Ausführungsform auch durchgeführt werden, wenn die Probenhalterung an die Mediumkammer und/oder die Suspensionskammer gekoppelt ist.

Gemäß einer Ausführungsform ist die Vorrichtung druckstabil von einem Unterdruck von ca. 1 x 10⁻¹⁰ mbar bis zu einem Überdruck von ca. 2 bar ausgebildet. Die angegebenen Werte sind Mindestwerte. Die Vorrichtung kann insbesondere auch bei tieferen Unterdrücken (stärker ausgeprägten Vakuum) oder bei höheren Überdrücken formstabil bleiben. Dazu kann die Vorrichtung zumindest teilweise aus Edelstahl und/oder einem geeigneten Kunststoff ausgebildet sein. Transparente Bereiche können aus Glas, Plexiglas und/oder einem transparenten Kunststoff ausgebildet sein. Die druckstabile Ausbildung der Vorrichtung ermöglicht eine Beaufschlagung der in die Vorrichtung eingebrachten Probe mit Über- und/oder Unterdruck, z.B. im Rahmen eines physikalischen Integritätstests und/oder für eine Druckbeaufschlagung mit einer kontaminierten Suspension bzw. einem kontaminierten Aerosol. Weiterhin kann die Mediumkammer mit oder ohne Kulturmedium unter Druck gesetzt werden, ebenso wie gegebenenfalls die Suspensionskammer, die mit einer Suspension gefüllt sein kann. Während der Druckbeaufschlagung bleibt die Probe sicher und dicht in der Probenhalterung eingespannt.

Gemäß einer Ausführungsform ist die Vorrichtung temperaturstabil von einer Temperatur von -50°C bis +100°C ausgebildet. Dazu kann die Vorrichtung zumindest z.B. teilweise aus Edelstahl und/oder einem geeigneten Kunststoff ausgebildet sein.

Gemäß einer Ausführungsform weist die Vorrichtung eine Elektrode zum Kontaktieren des Innenraums der Mediumkammer auf. Die Elektrode dient zum elektrischen und physikalischen Kontaktieren eines in der Mediumkammer angeordneten Kulturmediums. Gegebenenfalls kann eine weitere Elektrode zum Kontaktieren des Innenraums Suspensionskammer vorgesehen sein. Die Elektroden dienen zum Messen von elektrischen Strömen im Kulturmedium, in der Suspension und/oder zwischen dem Innenraum der Mediumkammer und dem Supsensionskammerinnenraum. Im Kulturmedium können dann Ströme auftreten, wenn das Kulturmedium kontaminiert ist. In diesem Fall dient die Elektrode der Detektion einer Kontamination. Dazu können elektrische Verbindungen der Elektrode an ein Strom- und/oder Spannungsmeßgerät vorgesehen sein, sowie weitere Elektroden.

Gemäß einer Ausführungsform sind Zuleitungen und/oder Ableitungen der Vorrichtung verschließbar ausgebildet. Die Zu- und Ableitungen dienen zum Zu- und Abfluss von Fluid und können schlauchförmig ausgebildet sein und zum Beispiel mittels einer außenliegenden Schlauchklemme abgeklemmt werden, die von außen an die Zuleitungen angesetzt werden kann. Als solche Zu- und Ableitungen können an der Vorrichtung zum Beispiel vorgesehen sein: Eine Mediumzuleitung zum Befüllen der Mediumkammer mit einem Kulturmedium, ein Mediumauslass zum Auslassen der Mediumkammer, eine Medienprobenentnahme zur Entnahme einer Probe aus der Mediumkammer, die Entlüftungsleitung, eine Suspensionszuleitung zum Einlassen einer Suspension in die Suspensionskammer, ein Suspensionsauslass zum Entleeren der Suspensionskammer, eine Suspensionsprobenentnahme zur Entnahme einer Probe aus der Suspensionskammer und/oder die Suspensionskammerentlüftungsleitung. Zum Verschließen der Zu- und Ableitungen können ein oder mehrere Leitungsverschlüsse vorgesehen sein.

Ein zweiter Aspekt betrifft ein Verfahren zur Durchführung eines Integritätstests an einer Probe von Einwegkomponenten mit den Schritten:
- Bereitstellen einer Mediumkammer mit einem nach außen begrenzten Innenraum;
- Anordnen der Probe in einer Probenhalterung derart, dass die Probe die Mediumkammer zumindest teilweise nach außen begrenzt;
- Ausrichten der Mediumkammer in einer Befüllposition derart, dass eine Entlüftungsleitung der Mediumkammer im Wesentlichen am höchsten Punkt des Innenraums der Mediumkammer angeordnet ist und
- Befüllen des Innenraums der Mediumkammer mit einem Kulturmedium bei geöffneter Entlüftungsleitung.

Anschließend kann ein Bakterienrückhaltetest (BCT) durchgeführt werden, bei dem das in die Mediumkammer eingefüllte Kulturmedium zur Überprüfung der Integrität der Probe dient.

Die Probe kann in einem Verfahrensschritt aus einer Einwegkomponente heraus getrennt werden. In der Befüllposition wird die Mediumkammer über die Entlüftungsleitung an ihrem höchsten Punkt im Bezugssystem der Erde entlüftet. Dadurch wird eine besonders günstige Möglichkeit zur Entlüftung der Mediumkammer bereitgestellt, bei der die Mediumkammer vollständig mit dem Kulturmedium ausgefüllt werden kann. Beim Befüllen des Innenraums der Mediumkammer mit dem Kulturmedium wird die Mediumkammer über die oben angeordnete Entlüftungsleitung entlüftet. Insbesondere kann die Entlüftungsleitung in der Befüllposition im Wesentlichen vertikal über der Probenhalterung angeordnet sein, wobei die Entlüftungsleitung an einer der Probenhalterung abgewandten Seite der Mediumkammer ausgebildet sein kann.

Gemäß einer Ausführungsform weist das Verfahren die Schritte auf:
- Ausrichten der Mediumkammer in einer Beaufschlagungsposition, in der die Probe mit einer geneigten Probenaußenfläche ausgerichtet ist und
- Beaufschlagen der Probe in der Beaufschlagungsposition von außen mit Bakterien und/oder Sporen.

Die Beaufschlagung kann mit einer Suspension und/oder mit einem Aerosol erfolgen, die bzw. das Bakterien und/oder Sporen enthält. In der Beaufschlagungsposition stellt die Probe eine Grenzfläche der Mediumkammer bereit, die in der Beaufschlagungsposition eine im Wesentlichen geneigt ausgerichtete Grenzfläche bzw. Beaufschlagungsfläche der Mediumkammer für die Beaufschlagung der Probe mit Bakterien und/oder Sporen bereitstellt. Auf die geneigt ausgerichteten Probenaußenfläche können die Bakterien besonders gut einwirken, was eine effiziente und starke Beanspruchung der Probe während des Integritätstests ermöglicht. Geneigt bedeutet hierbei, dass die Probenaußenfläche geneigt gegenüber einer horizontalen Ebene ist, also insbesondere vertikal ausgerichtet.

In einer Weiterbildung dieser Ausführungsform wird die Probe in der Suspensionsbefüllposition unmittelbar mit einem Bakterien- und/oder Sporenaerosol beaufschlagt, das sich dabei zumindest teilweise auf der Probe niederschlagen kann. Dazu kann die Mediumkammer mitsamt der Probe in einer Aerosolkammer positioniert werden, in der das Aerosol angeordnet ist. Auf der Probe niedergeschlagenes Aerosol ist besonders dazu geeignet, die Integrität der Probe zu überprüfen. Zusätzlich kann es vorgesehen sein, das Aerosol unter Überdruck vorzusehen, die Probe also mit dem Aerosol unter Überdruck zu beaufschlagen. Dazu kann die gesamte Aerosolkammer mit Überdruck beaufschlagt werden.

In einer alternativen oder zusätzlichen Weiterbildung dieser Ausführungsform wird das Verfahren mit den Schritten durchgeführt:
- Bereitstellen einer Suspensionskammer derart, dass ein Suspensionskammerinnenraum vom Innenraum der Mediumkammer zumindest teilweise nur durch die in der Probenhalterung angeordnete Probe getrennt ist, und
- Ausrichten der Vorrichtung in einer Suspensionsbefüllposition derart, dass eine Suspensionskammerentlüftungsleitung im Wesentlichen am höchsten Punkt des Suspensionskammerinnenraums angeordnet ist,
- Befüllen eines Suspensionskammerinnenraums in der Suspensionsbefüllposition mit einer Bakterien und/oder Sporensuspension bei geöffneter Suspensionskammerentlüftungsleitung.

Die Suspensionskammer ermöglicht eine Beaufschlagung der Probe mit einer kontaminierten Suspension. Dabei kann während des Integritätstests die Probe unmittelbar mit Suspension beaufschlagt werden. Beim Befüllen der Suspensionskammer mit der Suspension wird die Suspensionskammer nach oben hin über die Suspensionskammerentlüftungsleitung entlüftet, was eine vollständige Befüllung der Suspensionskammer ermöglicht. Die Suspensionskammerentlüftungsleitung kann an einer der Probenhalterung abgewandten Seite der Suspensionskammer angeordnet sein. In der Suspensionsbefüllposition kann die Mediumkammer so ausgerichtet sein, dass die Probe die Mediumkammer zumindest teilweise vertikal nach oben begrenzt.

Ein Aspekt betrifft die Verwendung einer Vorrichtung nach dem ersten Aspekt zum Durchführen eines Integritätstests an einer Probe von Einwegkomponenten gemäß einem Verfahren nach dem zweiten Aspekt.

Die Erfindung wird nachfolgend anhand von in Figuren gezeigten Ausführungsbeispielen näher erläutert. Es zeigen:
- Figur 1: in einer schematischen Darstellung eine Vorrichtung zur Durchführung eines Integritätstests mit einer auf eine Mediumkammer aufgesetzte Probe,
- Figur 2: in einer schematischen Darstellung die in Figur 1 dargestellte Vorrichtung mit aufgesetzter Suspensionskammer und
- Figuren 3A bis 3D: in jeweils einer schematischen Darstellung eine Ausführungsform einer Vorrichtung zur Durchführung eines Integritätstests mit einer auf eine Mediumkammer aufgesetzten Probe.

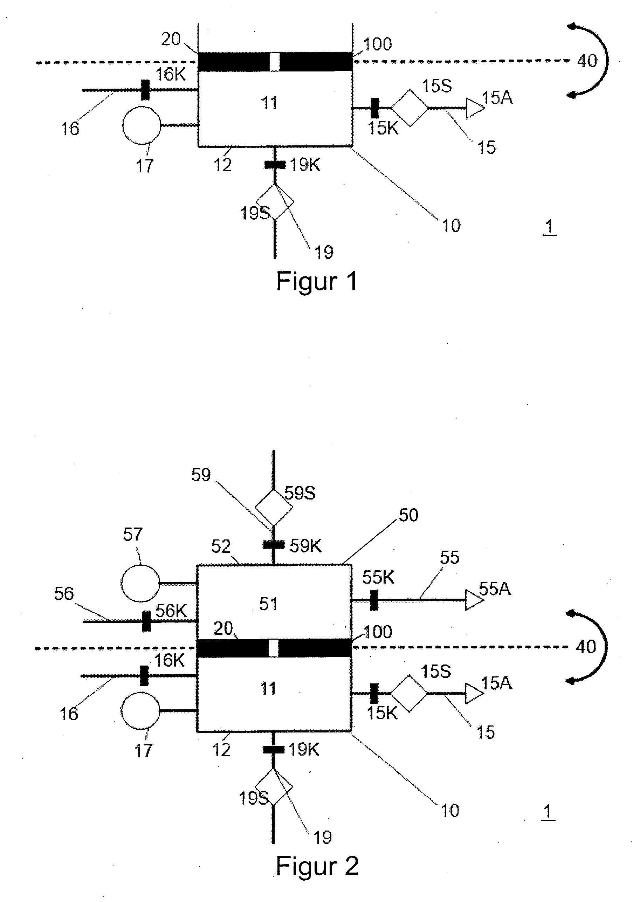

**Figur 1** zeigt in einer schematischen Darstellung eine Vorrichtung zur Durchführung eines Integritätstests an einer Probe von Einwegkomponenten. Die Vorrichtung 1 weist eine Mediumkammer 10 auf, deren Seitenwände 12 einen Innenraum der Mediumkammer, den sogenannten Mediumkammerinnenraum 11, definieren und umschließen. Eine Seitenwand der Mediumkammer 10 wird von einer Probe 100 gebildet. Die Probe 100 ist in einer Probenhalterung 20 angeordnet.

Als Probe kann eine scheibenförmige Probe einer Einwegkomponente für die biopharmazeutische Industrie verwendet werden. Die Probe kann aus einem ausgestanzten Stück Film und/oder Folie bestehen. Die Probe 100 ist im Wesentlichen flächig ausgebildet. Mit dieser Fläche begrenzt die in der Probenhalterung 20 angeordnete Probe 100 den Mediumkammerinnenraum 11 nach außen. Dabei kann die Probe 100 zum Beispiel scheibenförmig ausgebildet sein und dabei als ein Zylinderdeckel einer zylinderförmigen Mediumkammer 10 ausgebildet sein. In einer anderen Ausführungsform kann die Probe im Wesentlichen in Form einer quadratischen oder rechteckigen Fläche ausgebildet sein. Der Durchmesser bzw. die Diagonale der Probe 100 weist in diesen Fällen bevorzugt eine Größe von 20 mm bis 100 mm auf, insbesondere von 40 mm bis 70 mm, besonders bevorzugt von 50 mm auf. Die Volumenform der Mediumkammer 10 kann auf eine vorbestimmte Probenform abgestimmt sein, zum Beispiel, wie oben beschrieben, eine scheibenförmige, eine rechteckige, eine quadratische oder eine elliptische Form. Die Probe 100 kann in der Probenhalterung 20 so angeordnet werden, dass sie einen Deckel der Mediumkammer 10 bildet, zum Beispiel einen Zylinderdeckel einer zylinderförmigen Mediumkammer oder einen viereckigen Deckel einer kastenförmigen Mediumkammer.

Die Mediumkammer 10 ist dazu ausgebildet und vorgesehen, ein Kulturmedium wie zum Beispiel TSB aufzunehmen. Dazu weist die Mediumkammer 10 zumindest eine Mediumzuleitung 15 auf. Optional kann die Mediumkammer 10 einen Mediumauslass 16 aufweisen, aus dem ein Medium wie z.B. ein Kulturmedium aus der Mediumkammer 10 ausgelassen werden kann.

Die in den Figuren gezeigten Leitungen können in einer Ausführungsform als ein Bestandteil der Kammerwandung ausgebildet sein. Dabei benötigen die Leitungen in der radialen Ausrichtung wenig Bauraum und enthalten besonders wenig Kulturmedium. Hierbei findet ein bakterielles Wachstum zielgerichtet nur in der Mediumkammer und nicht (oder nur sehr begrenzt) in den angrenzenden Leitungen statt.

In der Seitenwand 12 der Mediumkammer 10, die der Probe 100 bzgl. des Mediumkammerinnenraums 11 gegenüberliegt, ist eine Entlüftungsleitung 19 angeordnet. Die Entlüftungsleitung 19 ist an der der Probenhalterung 20 abgewandten Seite der Mediumkammer 10 ausgebildet. Die Entlüftungsleitung 19 dient zur Entlüftung des Mediumkammerinnenraums 11. Die Entlüftungsleitung 19 ist mittels einer Entlüftungsleitungsklemme 19K verschließbar und öffenbar. Weiterhin ist in der Entlüftungsleitung 19 ein Entlüftungsleitungssterilfilter 19S angeordnet. Der Entlüftungsleitungssterilfilter 19S dient zum steril halten der Entlüftungsleitung 19, um eine unbeabsichtigte Kontamination der Mediumkammer 10 nach der Sterilisation zu verhindern.

Dazu sind können alle Leitungen entsprechende Sterilbarrieren aufweisen. Dies dient zur Vermeidung jeglicher vorheriger Kontamination bei der Probenvorbereitung, so dass lediglich eine bewusste Kontaminationen durch die Probe hindurch im Rahmen des Integritätstests stattfinden kann.

Sämtliche Zu- und Ableitungen der Vorrichtung 1 und insbesondere der Mediumkammer 10 sind als Sterilverbindungen ausgebildet. So weist die Mediumzuleitung 15 entweder einen Mediumzuleitungssterilfilter 15S, einen Mediumzuleitungsaseptikkonnektor 15A, ein Mediumzuleitungsseptum oder eine Kombination davon auf. Die Mediumzuleitung 15 weist weiterhin eine Mediumzuleitungsklemme 15K auf zum Verschließen und Öffnen der Mediumzuleitung 15. Auch der Mediumauslass 16 weist eine Mediumauslassklemme 16K auf zum Öffnen und Schließen des Mediumauslasses 16.

Die vorgenannten Klemmen können zum Beispiel als außenliegende Schlauchklemmen ausgebildet sein, die entweder von Hand durch einen Benutzer geöffnet und geschlossen werden können, oder aber automatisiert.

Die Probenhalterung 20 dient zur Halterung der Probe 100 derart, dass die Probe eine Seitenwand der Mediumkammer 10 ausbildet. Die Probenhalterung 20 kann zum Beispiel als Einspannvorrichtung ausgebildet sein. Die Probenhalterung 20 verbindet die Probe 100 mediendicht und steril mit der Mediumkammer 10, zum Beispiel über Flachdichtungen und/oder O-Ringe.

Die Mediumkammer 10 kann weiterhin eine Medienprobennahme 17 aufweisen, über die eine Medienprobe des im Innenraum 11 der Mediumkammer 10 befindlichen Kulturmediums entnommen werden kann. Die Medienprobennahme 17 kann zum Beispiel als Septum ausgebildet sein. Durch Entnahme einer Medienprobe des Kulturmediums kann eine Kontamination des in der Mediumkammer 10 befindlichen Kulturmediums überprüft werden.

Die Vorrichtung 1 kann vollständig oder teilweise aus Edelstahl und/oder Kunststoff ausgebildet sein. Ist die Vorrichtung aus Edelstahl ausgeführt, so sind in der Mediumkammer 10 bevorzugt zwei gegenüberliegende transparente Sichtfenster ausgebildet, über die eine Verfärbung bzw. Trübung des Kulturmediums detektiert werden kann. In der Kunststoffausführung der Vorrichtung 1 ist die Vorrichtung bevorzugt aus einem transparenten Kunststoff ausgebildet. Die jeweiligen transparenten Bereiche dienen zur einfachen Beobachtung eines bakteriellen Wachstums in der Mediumkammer 10. Die Vorrichtung ist aus einem sterilisierbaren Material ausgebildet, also einem Material, das einer Sterilisation durch Autoklavieren, Bedampfung mit Heißdampf, Gamma-Bestrahlung und/oder EtOH-Begasung standhält.

Die Vorrichtung 1 weist weiterhin eine Ausrichtvorrichtung 40 auf. Die Ausrichtvorrichtung 40 dient zur Ausrichtung der Mediumkammer 10 derart, dass die Entlüftungsleitung 19 an einem Punkt der Mediumkammer 10 ansetzt, der oberhalb der Probe 100 angeordnet ist. Die Ausrichtvorrichtung 40 kann insbesondere eine Schwenkachse aufweisen, die in Figur 1 gestrichelt dargestellt ist. Die Schwenkachse der Ausrichtvorrichtung 40 kann an der Probenhalterung 20 angreifen und die Vorrichtung 1 durch Verschwenken der Probenhalterung 20 und der damit verbundenen Mediumkammer 10 ausrichten.

Zum Befüllen der Mediumkammer 10 mit einem Kulturmedium wird die Mediumkammer 10 mittels der Ausrichtvorrichtung 40 über die Probenhalterung 20 geschwenkt. In dieser Befüllposition ist der Innenraum 11 der Mediumkammer 10 oberhalb der Probenhalterung 20 und somit auch über der Probe 100 angeordnet. Die Probe 100 bildet in der Befüllposition die Bodenfläche der Mediumkammer 10 aus. In der Befüllposition ist die Entlüftungsleitung 19 an der Seitenwand 12 der Mediumkammer ausgebildet, die das Dach bzw. den Deckel der Mediumkammer 10 bildet. In der Befüllposition wird die Mediumkammer 10 besonders einfach und blasenfrei mit einem Kulturmedium gefüllt, das über die Mediumzuleitung 15 in den Innenraum 11 der Mediumkammer 10 geleitet wird.

Die Seitenwand 12 der Mediumkammer 10, an der die Entlüftungsleitung 19 ansetzt, kann derart ausgebildet sein, dass sie sich zum Ansatzpunkt der Entlüftungsleitung 19 hin verjüngt. Diese verjüngende Form verbessert den Befüllvorgang der Mediumkammer 10 weiterhin und gewährleistet ein vollständiges Befüllen der Mediumkammer 10.

Die Ausrichtvorrichtung 40 ermöglicht ein Ausrichten der Vorrichtung 1 in unterschiedlichen Positionen. Insbesondere ermöglicht die Ausrichtvorrichtung 40 das Ausrichten der Vorrichtung 100 derart, dass die Probe 100 im Wesentlichen horizontal oder mit geneigter Probenaußenfläche ausgerichtet ist. Zur horizontalen Ausrichtung der Probe 100 gibt es zwei unterschiedliche Möglichkeiten: Einerseits die oben beschriebene Befüllposition, in der der Innenraum 11 der Mediumkammer 10 oberhalb der Probe 100 angeordnet ist und andererseits eine Suspensionsbefüllposition, die in den Figuren dargestellt ist und in der die Probe 100 oberhalb des Innenraums 11 der Mediumkammer 10 angeordnet ist.

**Figur 2** zeigt die in Figur 1 dargestellte Vorrichtung 1, auf die eine Suspensionskammer 50 aufgebracht wurde. Die Suspensionskammer 50 dient zur Aufnahme einer kontaminierten Suspension, in die Bakterien und/oder Sporen zum Überprüfen der Integrität der Probe 100 eingefüllt werden können.

Die Suspensionskammer 50 weist einen Suspensionskammerinnenraum 51 auf, der nach außen durch Suspensionskammerseitenwände 52 begrenzt ist. Eine Seitenwand der Suspensionskammer 50 wird durch die Probe 100 gebildet, die in der Probenhalterung 20 angeordnet ist. In der in Figur 2 dargestellten Position ist die Probe 100 als Grenzfläche zwischen dem Innenraum 11 der Mediumkammer 10 und dem Suspensionskammerinnenraum 51 der Suspensionskammer 50 ausgebildet. Die Suspensionskammer 50 und die Mediumkammer 10 sind an gegenüberliegenden Seitenflächen der Probe 100 angeordnet. Dabei ist der Mediumkammerinnenraum 11 an einer Probeninnenfläche der Probe 100 und der Suspensionskammerinnenraum 51 an einer Probenaußenfläche der Probe 100 angeordnet.

Sowohl die Suspensionskammer 50 als auch die Mediumkammer 10 sind lösbar mit der Probenhalterung 50 verbunden. Dazu kann die Probenhalterung 20 Verbindungselemente wie etwa ein Gewinde, Rastelemente oder Steckelemente aufweisen, die zum Verbinden mit der Mediumkammer 10 und/oder der Suspensionskammer 50 ausgebildet und vorgesehen sind. Entsprechend kann die Mediumkammer 10 und/oder die Suspensionskammer 50 komplementäre Verbindungselemente zum Verbinden mit den Verbindungselementen der Probenhalterung 20 aufweisen. Die Verbindung zwischen der Probenhalterung 20 und der Mediumkammer 10 ist mediendicht und sterilisierbar. Auch die Verbindung zwischen der Probenhalterung 20 und der Suspensionskammer 50 kann mediendicht und sterilisierbar ausgebildet sein.

Die zunächst lösbare Verbindung zwischen Probenhalterung und Mediumkammer und/oder Supsensionskammer kann permanent fixiert werden, z.B. durch ein Verkleben und/oder Verspritzen. Eine permanent fixierte Verbindung reduziert z.B. die Gefahr einer unbeabsichtigten Kontamination.

Alternativ könnten die Mediumkammer und die Suspensionskammer auch unmittelbar miteinander verbindbar ausgebildet sein. In dieser Ausführungsform lässt sich die in der Probenhalterung angeordnete Probe so zwischen die beiden Kammern einführen, dass die Probe eine Grenzfläche zwischen den jeweiligen Innenräumen ausbildet.

Wie in Figur 2 gezeigt kann die Suspensionskammer 50 im Wesentlichen spiegelbildlich zur Mediumkammer 10 ausgebildet sein und entsprechende Bemaßungen und Formen aufweisen.

In der Suspensionskammerseitenwand 52, die gegenüber der Probenhalterung 20 angeordnet ist, weist die Suspensionskammer 50 eine Suspensionskammerentlüftungsleitung 59 auf mit einer Suspensionskammerentlüftungsleitungsklemme 59K zum Verschließen und Öffnen der Suspensionskammerentlüftungsleitung 59. Weiterhin weist die Suspensionskammerentlüftungsleitung 59 einen Suspensionskammerentlüftungsleitungssterilfilter 59S auf zur Sterilisation der Leitung.

Analog zur Mediumkammer 10 kann die Suspensionskammer 50 eine Suspensionszuleitung 55 aufweisen mit einem Suspensionszuleitungsaseptikkonnektor 55A und einer Suspensionszuleitungsklemme 55K. Weiterhin kann die Suspensionskammer 50 mit einem Suspensionsauslass 56 zum Auslassen der Suspension aus dem Suspensionskammerinnenraum 51 verbunden sein, der mittels einer Suspensionsauslassklemme 56K verschließbar und öffenbar ist. Eine Suspensionsprobennahme 57 dient zur Entnahme einer Probe der Suspension und kann optional vorgesehen sein. Die Suspensionsprobennahme 57 kann als Septum ausgebildet sein.

In der oben beschriebenen Suspensionsbefüllposition der Vorrichtung 1, in der die Vorrichtung 1 in Figur 2 gezeigt ist, kann der Suspensionskammerinnenraum 51 über die Suspensionszuleitung 55 mit einer Suspension vollständig gefüllt werden. In der Suspensionsbefüllposition ist die Suspensionskammerentlüftungsleitung 59 am oberen Ende der Suspensionskammer 50 angeordnet, was eine vollständige Entlüftung des Suspensionskammerinnenraums 51 ermöglicht. Die Suspensionskammerseitenwand 52, in der die Suspensionskammerentlüftungsleitung 59 ansetzt, kann mit einem sich verjüngenden Querschnitt ausgebildet sein, der sich hin zum Ansatzpunkt der Suspensionskammerentlüftungsleitung 59 verjüngt.

Die Entlüftungsleitung 19 der Mediumkammer 10 und die Suspensionskammerentlüftungsleitung 59 sind an einander gegenüberliegenden Enden der Vorrichtung 1 angeordnet. Sämtliche weiteren Zu- und Ableitungen können auch an seitlichen Seitenwänden der Vorrichtung angeordnet sein. Um die horizontalen Ausmaße der Vorrichtung 1 zu begrenzen, können sämtliche Zu- und Ableitungen (anders als in den Figuren dargestellt) an den beiden Seitenwänden ansetzen, an denen die Entlüftungsleitung 19 und die Suspensionskammerentlüftungsleitung 59 ansetzen, also an den Seitenwänden, die die Vorrichtung 1 in der Befüllposition und in der Suspensionsbefüllposition an ihren vertikalen Enden begrenzen.

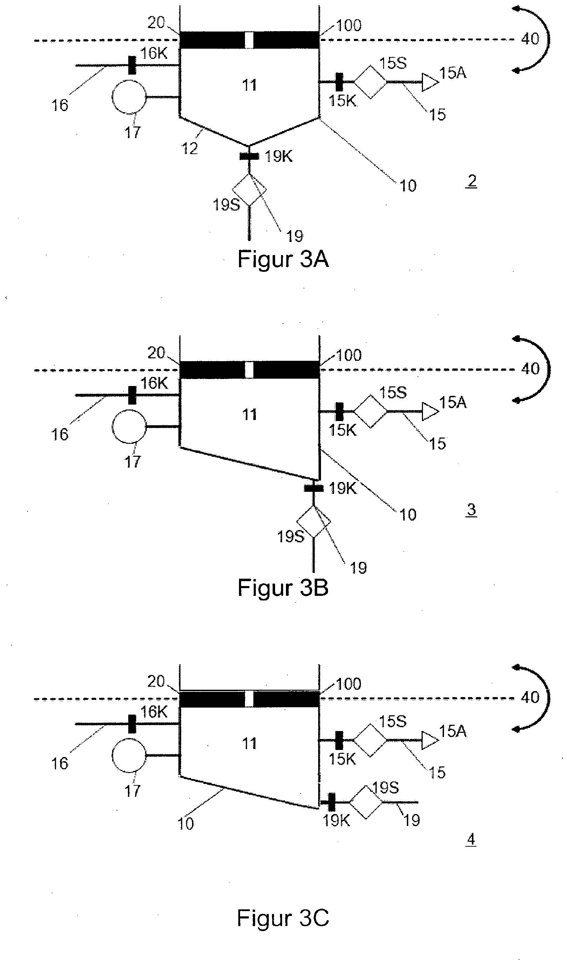

**Figur 3A** zeigt eine Ausführungsform einer Vorrichtung 2, bei der die Seitenwand 12 der Mediumkammer 10, die der Probenhalterung 20 abgewandt ist, mit einem sich mit zunehmendem Abstand von der Probenhalterung 20 verjüngenden Querschnitt ausgebildet ist. Die Entlüftungsleitung 19 ist an der Position der der Probenhalterung 20 abgewandten Seite der Medienkammer 10 angeordnet, die den geringsten Querschnitt aufweist. Die Entlüftungsleitung 19 ist in der in Figur 3A gezeigten Vorrichtung 2 zentral an der der Probenhalterung 20 abgewandten Seite der Medienkammer 10 angeordnet.

**Figur 3B** zeigt eine Ausführungsform einer Vorrichtung 3, bei der die Seitenwand 12 der Mediumkammer 10, die der Probenhalterung 20 abgewandt ist, wie bei Vorrichtung 2 mit einem sich mit zunehmendem Abstand von der Probenhalterung 20 verjüngenden Querschnitt ausgebildet ist. Bei der Vorrichtung 3 ist Entlüftungsleitung 19 dezentral an der der Probenhalterung 20 abgewandten Seite der Medienkammer 10 angeordnet.

Bei den in den Figuren 3A und 3B gezeigten Vorrichtungen 2 und 3 ist die Entlüftungsleitung 19 so angeordnet, dass sie im Wesentlichen parallel zu einer Normalen auf die Probe 100 von der Medienkammer 10 weg verläuft und ausgerichtet ist.

**Figur 3C** zeigt in einem Ausführungsbeispiel eine Vorrichtung 4, bei der diejenige Seitenwand 12 der Mediumkammer 10, die der Probenhalterung 20 abgewandt ist, auch mit einem sich mit zunehmendem Abstand von der Probenhalterung 20 verjüngenden Querschnitt ausgebildet ist, ähnlich wie bei Vorrichtung 3. Im Unterschied zu Vorrichtung 3 setzt die Entlüftungsleitung 19 zwar an dem Punkt des Innenraums 11 der Mediumkammer 10 an, der am weitesten von der Probenhalterung 20 und somit der Probe 100 entfernt ist, verläuft aber nicht senkrecht, sondern im Wesentlichen parallel zur Probe 100.

Alternativ könnte die Entlüftungsleitung 19 auch so angeordnet sein, dass sie schräg von der Probe 100 und der Mediumkammer 10 weg verläuft.

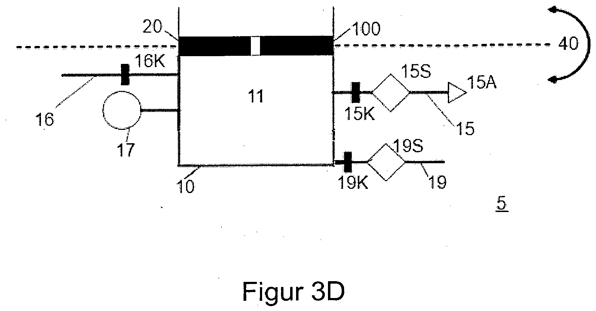

**Figur 3D** zeigt in einem Ausführungsbeispiel eine Vorrichtung 5, bei der die der Probenhalterung 20 gegenüberliegende Seitenwand 12 der Mediumkammer 10 im Wesentlichen parallel zur Probe ausgebildet ist. Die Entlüftungsleitung 19 setzt dabei an einer Seitenwand der Mediumkammer 10 an, die selbst nicht der Probenhalterung 20 gegenüber liegt, sondern an die Probenhalterung 20 angrenzt. Hierbei setzt die Entlüftungsleitung 19 unmittelbar benachbart zur gegenüberliegenden Seitenwand 12 der Mediumkammer 20 an. Auch bei Vorrichtung 5 ist die Entlüftungsleitung 19 somit an der der Probenhalterung 20 abgewandten Seite der Mediumkammer 10 ausgebildet.

Die in den Figuren 3A bis 3D gezeigten Vorrichtungen 2 bis 5 sind jeweils ohne Suspensionskammer 50 gezeigt. Die Vorrichtungen 2 bis 5 können jeweils Verbindungselemente zum verbinden mit einer Suspensionskammer 50 aufweisen, die z.B. spiegelverkehrt zur Medienkammer 10 bezüglich der in der Probenhalterung 20 angeordneten Probe 100 ausgebildet sein kann, wie in Fig. 2 für Vorrichtung 1 gezeigt.

Mit der Vorrichtung kann ein Integritätstest einer Probe durchgeführt werden, und zwar insbesondere mit dem nachfolgend beschriebenen Verfahren:

### Verfahren zur Durchführung eines Inteqritätstests an einer Probe von Einwegkomponenten

Ein Verfahren zur Durchführung eines Integritätstests an einer Probe von Einwegkomponenten kann mit der in den Figuren gezeigten Vorrichtung 1 durchgeführt werden. Das Verfahren kann mit einigen oder allen der nachfolgend aufgelisteten Verfahrensschritte durchgeführt werden. Einzelne Verfahrensschritte können weggelassen werden. Zusätzlich kann das Verfahren ein Durchführen von weiteren Verfahrensschritte beinhalten. Die Verfahrensschritte können dabei z.B. in der nachfolgend aufgeführten Reihenfolge durchgeführt werden. Einzelne Verfahrensschritte können jedoch auch vorab bzw. später durchgeführt werden.
- Schritt a:: Einbringen der Probe 100 in die Probenhalterung 20.
- Schritt b:: Durchführen eines physikalischen Integritätstests.

Der physikalische Integritätstest kann als ein Teiltest des insgesamt durchzuführenden Integritätstests durchgeführt werden. Beim physikalischen Integritätstest kann die Probe auf eine physikalische Belastbarkeit hin überprüft werden und/oder auf ein vorhandenes Leck. Der physikalische Integritätstest der Probe 100 kann ohne Mediumkammer 10 und ohne Suspensionskammer 50 durchgeführt werden, also lediglich an der in die Probenhalterung 20 eingebrachten Probe 100. Alternativ kann der physikalische Integritätstest mit an der Probenhalterung 20 befestigten Mediumkammer 10 und/oder mit an der Probenhalterung 20 befestigten Suspensionskammer 50 durchgeführt werden. Dabei kann die Mediumkammer 10 mit Ausnahme der Entlüftungsleitung 19 dicht verschlossen sein. Sämtliche Leitungen an der Suspensionskammer sind dabei für Gasströme geöffnet, ggf. über ihre jeweils zugehörige Sterilbarriere. Dabei kann zur Durchführung des physikalischen Integritätstest ein Messgerät an die Entlüftungsleitung 19 und/oder an die Suspensionskammerentlüftungsleitung 59 angeschlossen werden. Als mögliche Testverfahren des physikalischen Integritätstests kann ein Druckabfallmessverfahren oder ein Gasdetektionsverfahren eingesetzt werden, letzteres z.B. mit Kohlendioxid, Formiergas, Stickstoff, SF6 und/oder Helium als Testgas.
- Schritt c:: Bereitstellen der Mediumkammer 10 mit nach außen begrenzten Innenraum 11.
- Schritt d:: Anordnen der Probe 100 in der Probenhalterung 20 derart, dass die Probe 100 die Mediumkammer 10 zumindest teilweise nach außen begrenzt.

Dabei kann die Mediumkammer 10 mit der Probenhalterung 20 verbunden werden, falls diese nicht vorher schon miteinander verbunden waren.
- Schritt e:: Sterilisation der Vorrichtung 1, z.B. mittels Autoklavieren, Bedampfung mit Heißdampf, Gamma-Bestrahlung und/oder EtOH-Begasung.
- Schritt f:: Ausrichten der Mediumkammer 10 in der Befüllposition derart, dass die Entlüftungsleitung 19 der Mediumkammer 10 im Wesentlichen am höchsten Punkt des Innenraums 11 der Mediumkammer 10 angeordnet ist.

In der Befüllposition ist der Innenraum 11 der Mediumkammer 10 im Wesentlichen vertikal über der Probe 100 angeordnet, die in der Befüllposition zumindest teilweise den Boden der Mediumkammer 10 bildet. Die Entlüftungsleitung 19 ist dabei im Wesentlichen vertikal über der Probenhalterung 20 angeordnet, wobei die Entlüftungsleitung 19 an der der Probenhalterung 20 abgewandten Seite der Mediumkammer 10 angeordnet ist.
- Schritt g:: Befüllen des Innenraums 11 der Mediumkammer 10 mit einem Kulturmedium bei geöffneter Entlüftungsleitung 19.

Dabei kann die Mediumkammer 10 soweit mit dem Kulturmedium gefüllt werden, bis das Kulturmedium bis über die Entlüftungsleitungsklemme 19K steigt. Anschließend können sämtliche Absperrvorrichtungen an allen Zu- und Ableitungen geschlossen werden, also insbesondere die Entlüftungsleitungsklemme 19K, die Mediumzuleitungsklemme 15K und die Mediumauslassklemme 16K. Dadurch wird die Mediumkammer 10 vollständig mit dem Kulturmedium gefüllt.

Das Kulturmedium kann über vorbereitete Beutel und/oder Spritzen an die Mediumzuleitung 15 angeschlossen werden. Diese Ankopplung des Kultumediums an die Mediumzuleitung 15 kann dabei vor der Sterilisation gemäß Verfahrensschritt e erfolgen.
- Schritt h:: Durchführen einer ersten Inkubationsphase.

Dabei kann die Vorrichtung 1 unter geeigneten Kultivierungsbedingungen für einen vorbestimmten Zeitraum beobachtet und auf mikrobiologisches Wachstum untersucht werden. Der vorbestimmte Zeitraum der ersten Inkubationsphase kann z.B. mindestens sieben Tage betragen. Die Überprüfung während der ersten Inkubationsphase kann z.B. durch Probennahme an der Medienprobenentnahme 17 erfolgen, oder durch Beobachtung durch einen transparenten Bereich der Seitenwand 12 der Mediumkammer 10. Dabei wird überprüft, ob ohne besondere Beaufschlagung mit Bakterien und/oder Sporen bereits ein mikrobiologisches Wachstum des Kulturmediums in der Mediumkammer 10 stattfindet, was auf eine Kontamination des Kulturmediums durch die Probe 100 hindurch hinweist, oder auf eine unzureichende Sterilisation der Vorrichtung.

Das Verfahren wird an dieser Stelle in einer Ausführungsform beschrieben, bei der die Vorrichtung 1 zusammen mit der Suspensionskammer 50 verwendet wird. Alternativ kann das Verfahren ab diesem Verfahrensschritt h auch ohne Verwendung einer Suspensionskammer 50 durchgeführt werden, was weiter unter in einer alternativen Ausführungsform des Verfahrens beschrieben wird.
- Schritt i:: Bereitstellen der Suspensionskammer 50 derart, dass der Suspensionskammerinnenraum 51 vom Innenraum 11 der Mediumkammer 10 zumindest teilweise nur durch die in der Probenhalterung 20 angeordnete Probe 100 getrennt ist.

Falls die Suspensionskammer 50 zuvor noch nicht mit der Vorrichtung 1 verbunden war, kann das Bereitstellen der Suspensionskammer ein Verbinden der Suspensionskammer 50 mit der Vorrichtung 1 beinhalten, insbesondere ein Verbinden mit der Probenhalterung 20 und/oder der Mediumkammer 10. Bei einer nachträglich angebrachten Suspensionskammer sollte diese zusätzlich separat sterilisiert worden sein, z.B. in einem Tyvekbeutel mit Sterilbarrriere, um die Suspensionskammer bei der Zwischenlagerung nicht zu kontaminieren.

Die Handhabung aller Vorrichtungsteile bis zum Anbringen der Suspensionkammer nach der Sterilisation wird bevorzugt unter einer Cleanbench durchgeführt. Ansonsten könnte die Kontamination mit einem anderen Organismus als dem Testorganismus erfolgen. Hierbei könnte ein zusätzlicher Nachweis des gefunden Testorganismus durchgeführt werden.

Der Suspensionskammerinnenraum 51 ist in der Suspensionsbefüllposition oberhalb der Probe 100 angeordnet.
- Schritt j:: Ausrichten der Vorrichtung 1 in einer Suspensionsbefüllposition derart, dass eine Suspensionskammerentlüftungsleitung 59 im Wesentlichen am höchsten Punkt des Suspensionskammerinnenraums 51 angeordnet ist.

In der Suspensionsbefüllposition kann der Innenraum 11 der Mediumkammer 10 im Wesentlichen unterhalb der Probe 100 angeordnet sein. Dabei kann die Probe 100 die Mediumkammer 10 zumindest teilweise vertikal nach oben begrenzen.
- Schritt k:: Befüllen des Suspensionskammerinnenraums 51 in der Suspensionsbefüllposition mit einer Bakterien- und/oder Sporensuspension bei geöffneter Suspensionskammerentlüftungsleitung 59.

Dabei kann die Suspensionskammer 50 bis über die Suspensionskammerentlüftungsleitungsklemme 59K hinaus mit der Suspension gefüllt werden. Anschließend können alle Absperrvorrichtungen an allen Zu- und Ableitungen geschlossen werden, also insbesondere die Suspensionskammerentlüftungsleitungsklemme 59K, die Suspensionszuleitungsklemme 55K und die Suspensionsauslassklemme 56K. Dadurch wird die Suspensionskammer 50 vollständig mit der Suspension gefüllt. Die Suspensionskammerentlüftungsleitung 59 kann an der der Probenhalterung 20 abgewandten Seite der Suspensionskammer 50 angeordnet sein.

Die Suspension kann über vorbereitete Beutel und/oder Spritzen an die Suspensionszuleitung 55 angeschlossen werden. Die Suspension kann als Bakterien- und/oder Sporenhaltige Suspension ausgebildet sein für einen Suspensions-BCT.
- Schritt I:: Ausrichten der Probe 100 in einer vorbestimmten Beaufschlagungsposition.

Die vorbestimmte Beaufschlagungsposition kann mit der Suspensionsbefüllposition übereinstimmen. In der Beaufschlagungsposition kann die Probe 100 alternativ im Wesentlichen vertikal ausgerichtet sein.
- Schritt m:: Halten der Probe 100 für eine vorbestimmte Einwirkzeit in der Beaufschlagungsposition derart, dass die Suspensionslösung flächig auf die Probe 100 einwirkt.

Die vorbestimmte Einwirkzeit kann dabei von 10 Minuten bis 200 Minuten betragen. Hierbei kann die Vorrichtung 1 optional einseitig mit einem Druck beaufschlagt werden, z.B. mit einem Druck von bis zu 2 bar. Die einseitige Druckbeaufschlagung kann z.B. entweder auf Seite der Mediumkammer 10 oder auf Seite der Suspensionskammer 50 über die jeweilige Entlüftungsleitung 19 bzw. 59 erfolgen.

Weiterhin kann während des Verfahrensschritts m ein (z.B. zusätzlicher) physikalischer Integritätstest durchgeführt werden, z.B. durch Messen eines Durchgangswiderstands und/oder der Leitfähigkeit über jeweils eine Elektrode, die den Innenraum 11 der Mediumkammer 10 kontaktiert und eine Elektrode, die den Suspensionskammerinnenraum 51 kontaktiert.
- Schritt n:: Ausrichten der Vorrichtung 1 mittels der Ausrichtvorrichtung 40 derart, dass der Suspensionsauslass 56 am tiefsten Punkt des Suspensionskammerinnenraums 51 angeordnet ist.
- Schritt o:: Vollständiges Entleeren der Suspensionskammer 50.
- Schritt p:: Spülen der Suspensionskammer 50, z.B. durch überströmendes Spülen mit Reinstwasser.
- Schritt q:: Vollständiges Entleeren der Suspensionskammer 50.
- Schritt p:: Optionales Trocknen der Suspensionskammer 50, z.B. durch Durchströmen mit Druckluft.
- Schritt r:: Durchführen einer zweiten Inkubationsphase.

Dabei kann die Vorrichtung 1 unter geeigneten Kultivierungsbedingungen für einen vorbestimmten Zeitraum beobachtet und auf mikrobiologisches Wachstum untersucht werden. Der vorbestimmte Zeitraum der zweiten Inkubationsphase kann z.B. von sieben Tage bis 21 Tage betragen. Die Überprüfung während der zweiten Inkubationsphase kann z.B. durch Probennahme an der Medienprobenentnahme 17 erfolgen und/oder durch Beobachtung durch einen transparenten Bereich der Seitenwand 12 der Mediumkammer 10. Dabei wird überprüft, ob Bakterien und/oder Sporen ein mikrobiologisches Wachstum des Kulturmediums in der Mediumkammer 10 bewirken, was auf eine Kontamination des Kulturmediums durch die Probe 100 hindurch hinweist. Zur Schlussüberprüfung kann das Kulturmedium auch vollständig aus der Mediumkammer 10 ausgelassen werden und anschließend überprüft werden.

Bei Feststellung einer Kontamination der Mediumkammer hat die Probe den Suspensions-BCT als Teiltest des Integritätstests nicht bestanden.

### Alternative Ausführungsform des Verfahrens

Alternativ kann das Verfahren anstelle mit den Verfahrensschritten i bis r anschließend an die ersten Inkubationsphase (Schritt h) mit einigen oder allen der folgenden Verfahrensschritten weitergeführt werden:
- Schritt s:: Ausrichten der Probe 100 in einer vorbestimmten Beaufschlagungsposition.

Die vorbestimmte Beaufschlagungsposition kann mit der Suspensionsbefüllposition übereinstimmen. In der Beaufschlagungsposition kann die Probe 100 alternativ im Wesentlichen vertikal ausgerichtet sein.
- Schritt t:: Einbringen der Vorrichtung 1 in eine Aerosolkammer.

Die Aerosolkammer ist zur Aerosolisierung im Rahmen eines Aerosol-BCT vorgesehen und ausgebildet und zusätzlich dazu, die Vorrichtung 1 aufzunehmen (gegebenenfalls auch mehrere Vorrichtungen 1). Die Vorrichtung 1 kann dabei ohne Suspensionskammer 50 verwendet werden, also wie in der in Figur 1 schematisch gezeigten Ausführungsform. Ggf. kann vor dem Schritt t die Suspensionskammer 50 von der Vorrichtung 1 gelöst werden.
- Schritt u:: Halten der Probe 100 für eine vorbestimmte Einwirkzeit in der Beaufschlagungsposition derart, dass ein in der Aerosolkammer befindliches Aerosol flächig auf die Probe 100 einwirkt.

Die vorbestimmte Einwirkzeit kann dabei von 10 Minuten bis 200 Minuten betragen. Während der vorbestimmten Einwirkzeit weist das in der Aerosolkammer befindliche Aerosol eine vorbestimmte Konzentration auf. Ggf. wird die Probe deswegen zusätzlich zur vorbestimmten Einwirkzeit noch während einer Anfangsphase in der Aerosolkammer gehalten, in der eine vorbestimmte Minimalkonzentration des Aerosols bereitgestellt wird. Das Aerosol ist zur Durchführung eines BCT als Bakterien- und/oder Sporenaerosol ausgebildet.

Dabei kann die Probe 100 in der Beaufschlagungsposition z.B. horizontal mit der Probe nach oben ausgerichtet sein, oder in einer alternativen Ausführungsform im Wesentlichen vertikal mit einer Neigung zur Horizontalen.

Hierbei kann die Vorrichtung 1 optional einseitig mit einem Druck beaufschlagt werden, z.B. mit einem Druck von bis zu 2 bar. Die einseitige Druckbeaufschlagung kann z.B. über die Entlüftungsleitung 19 der Mediumkammer 10 erfolgen.
- Schritt v:: Entnehmen der Vorrichtung 1 mitsamt der Probe 100 aus der Aerosolkammer.

Alternativ kann die Aerosolkammer hierbei auch vom Aerosol gereinigt werden.
- Schritt w:: Spülen der Probe 100, z.B. durch überströmendes Spülen mit Reinstwasser.
- Schritt x:: Optionales Trocknen der Probe 100 und ggf. der Suspensionskammer 50, z.B. durch Durchströmen mit Druckluft.
- Schritt y: Durchführen einer zweiten Inkubationsphase.

Wie im ersten Ausführungsbeispiel des Verfahrens beschrieben, kann die Vorrichtung 1 dabei unter geeigneten Kultivierungsbedingungen für einen vorbestimmten Zeitraum beobachtet und auf mikrobiologisches Wachstum untersucht werden. Der vorbestimmte Zeitraum der zweiten Inkubationsphase kann z.B. von sieben Tagen bis 21 Tage betragen. Die Überprüfung während der zweiten Inkubationsphase kann z.B. durch Probennahme an der Medienprobenentnahme 17 erfolgen, durch Beobachtung durch einen transparenten Bereich der Seitenwand 12 der Mediumkammer 10 und/oder durch Spannungs- oder Strommessung über eine Messelektrode. Dabei wird überprüft, ob Bakterien und/oder Sporen ein mikrobiologisches Wachstum des Kulturmediums in der Mediumkammer 10 bewirken, was auf eine Kontamination des Kulturmediums durch die Probe 100 hindurch hinweist. Zur Schlussüberprüfung kann das Kulturmedium auch vollständig aus der Mediumkammer 10 ausgelassen werden und anschließend überprüft werden.

Während der zweiten Inkubationsphase kann auf der Probenaußenseite ein sterilisierter Deckel angebracht werden, der nach dem Trocknen angebracht wird. Dabei wird das Risiko einer Kreuzkontamination bei der weiteren Handhabung minimiert. Der Deckel kann z.B. auch unter einer Cleanbench angebracht werden.

Bei Feststellung einer Kontamination der Mediumkammer hat die Probe den Aerosol-BCT als Teiltest des Integritätstests nicht bestanden.

### Weitere Alternativen des Verfahrens

Im Anschluss an die zweite Inkubationsphase (gemäß Verfahrensschritt r bzw. Verfahrensschritt y) kann die Mediumkammer 10 vollständig entleert werden, z.B. analog zu den Verfahrensschritten n bis p zum Entleeren der Suspensionskammer 50.

Die zuvorbeschriebenen Verfahren können auf vielfältige Art variiert werden. So kann in einigen Ausführungsformen die erste Inkubationsphase gemäß Verfahrensschritt h entfallen. In diesem Fall wird nicht mittels der ersten Inkubationsphase die Sterilisation der Vorrichtung 1 und die mediendichte Befestigung der Probe 100 an der Mediumkammer 10 verifiziert.

Weiterhin könnte sowohl ein Suspensions-BCT gemäß den Verfahrensschritten i bis r als auch ein Aerosol-BCT gemäß den Verfahrensschritten s bis y in einer Kombination durchgeführt werden.

Die Verfahren können seriell oder parallel mit einer statistisch relevanten Probenzahl unter gleichen Bedingungen durchgeführt werden, also z.B. mit dem gleichen Kulturmedium, mit den gleichen Bakterien und/oder Sporen in der Suspension und/oder im Aerosol, bei identischen Inkubationsbedingungen etc.

Das Verfahren kann spätestens ab Sterilisation der Vorrichtung gemäß Verfahrensschritt e unter aseptischen Laborbedingungen als Cleanbench durchgeführt werden.

Die Ergebnisse des physikalischen Integritätstests gemäß Verfahrensschritt b (und/oder ggf. nach Verfahrensschritt m) können mit den Ergebnissen des BCT-Tests korreliert werden, die nach den zweiten Inkubationsphasen gemäß den Verfahrensschritten r und/oder y vorliegen.

Zur Bewertung der Detektionsgrenzen des physikalischen Integritätstests in Korrelation mit dem Bakterienrückhaltetest kann das Verfahren auch mit Proben mit vorbestimmten Fehlstellen durchgeführt werden. Die Fehlstellen können dabei im Wesentlichen als Löcher mit einem Durchmesser von 0,1 µm bis 100 µm ausgebildet sein.

### Bezugszeichenliste

- 1, 2, 3: Vorrichtung
- 4, 5: Vorrichtung
- 10: Mediumkammer
- 11: Mediumkammerinnenraum
- 12: Seitenwand
- 15: Mediumzuleitung
- 15A: Mediumzuleitungsaseptikkonnektor
- 15K: Mediumzuleitungsklemme
- 15S: Mediumzuleitungssterilfilter
- 16: Mediumauslass
- 16K: Mediumauslassklemme
- 17: Medienprobenentnahme
- 19: Entlüftungsleitung
- 19K: Entlüftungsleitungsklemme
- 19S: Entlüftungsleitungssterilfilter
- 20: Probenhalterung
- 40: Ausrichtvorrichtung
- 50: Suspensionskammer
- 51: Suspensionskammerinnenraum
- 52: Suspensionskammerseitenwand
- 55: Suspensionszuleitung
- 55A: Suspensionszuleitungsaseptikkonnektor
- 55K: Suspensionszuleitungsklemme
- 56: Suspensionsauslass
- 56K: Suspensionsauslassklemme
- 57: Suspensionsprobenentnahme
- 59: Suspensionskammerentlüftungsleitung
- 59K: Suspensionskammerentlüftungsleitungsklemme
- 59S: Suspensionskammerentlüftungsleitungssterilfilter
- 100: Probe

## Patentansprüche

1. Vorrichtung zur Durchführung eines Integritätstests an einer Probe von Einwegkomponenten mit
- einer Mediumkammer (10) mit einem nach außen begrenzten Innenraum (11) zur Aufnahme eines Kulturmediums;
- einer Probenhalterung (20), in der die Probe (100) so anordenbar ist, dass die Probe (100) die Mediumkammer (10) zumindest teilweise nach außen begrenzt; und
- einer Ausrichtvorrichtung (40);
wobei
- die Mediumkammer (10) eine Entlüftungsleitung (19) aufweist, die an einer der Probenhalterung (20) abgewandten Seite der Mediumkammer (10) verschließbar und öffenbar angeordnet ist;
- die Mediumkammer (10) mittels der Ausrichtvorrichtung (40) in einer Befüllposition ausrichtbar ist, in der
- die Entlüftungsleitung (19) der Mediumkammer (10) im Wesentlichen am höchsten Punkt des Innenraums (11) der Mediumkammer (10) angeordnet ist
- die Mediumkammer (10) über eine Mediumzuleitung (15) der Mediumkammer (10) mit dem Kulturmedium befüllbar ist und
- in der die Probenhalterung (20) vertikal unter der Entlüftungsleitung (19) angeordnet ist; und
- die Mediumkammer (10) mittels der Ausrichtvorrichtung (40) zudem in einer Suspensionsbefüllposition und/oder in einer Beaufschlagungsposition ausrichtbar ist, in der die Probenhalterung (20) vertikal oberhalb der Entlüftungsleitung (19) angeordnet ist.

2. Vorrichtung nach Anspruch 1, wobei die Mediumkammer (10) mittels der Ausrichtvorrichtung (40):
- in der Suspensionsbefüllposition so ausrichtbar ist, dass die Probenhalterung (20) vertikal oberhalb der Entlüftungsleitung (19) angeordnet ist und
- zudem in einer Beaufschlagungsposition so ausrichtbar ist, dass die Probe mit ihrer Probenaußenfläche zur Horizontalen geneigt angeordnet ist.

3. Vorrichtung nach Anspruch 1 oder 2, wobei die Entlüftungsleitung (19) in der Befüllposition der Mediumkammer (10) am vertikal höchsten Punkt der Mediumkammer (10) angeordnet ist und die Probenhalterung (20) am vertikal tiefsten Punkt der Mediumkammer (10) angeordnet ist.

4. Vorrichtung nach einem der vorangegangenen Ansprüche, mit einem Verbindungselement zum Verbinden der Mediumkammer (10) mit einer Suspensionskammer (50) derart, dass die in der Probenhalterung (20) angeordnete Probe (100) eine Grenzfläche zwischen dem Innenraum der Mediumkammer (10) und einem Suspensionskammerinnenraum (51) der Suspensionskammer (50) bereitstellt.

5. Vorrichtung nach Anspruch 4, mit einer Suspensionskammerentlüftungsleitung (59) der Suspensionskammer (50), die an einer der Probenhalterung (20) abgewandten Seiten der Suspensionskammer (50) angeordnet ist.

6. Vorrichtung nach einem der vorangegangenen Ansprüche, wobei in einer Seitenwand (12) der Mediumkammer (10) zumindest ein transparenter Bereich ausgebildet ist.

7. Vorrichtung nach einem der vorangegangenen Ansprüche, wobei die Probenhalterung (20) in einer lösbaren Verbindung mit der Mediumkammer (10) angeordnet ist.

8. Vorrichtung nach einem der vorangegangenen Ansprüche, wobei die Vorrichtung (1; 2; 3; 4; 5) druckstabil von einem Unterdruck von ca. 1*10⁻¹⁰ mbar bis zu einem Überdruck von ca. 2 bar ausgebildet ist.

9. Vorrichtung nach einem der vorangegangenen Ansprüche, mit zumindest einer Elektrode zum Kontaktieren des Innenraums (11) der Mediumkammer (10).

10. Vorrichtung nach einem der vorangegangenen Ansprüche, wobei Zu- und Ableitungen (15, 16, 17, 19, 55, 56, 57, 59) der Vorrichtung (1; 2; 3; 4; 5) verschließbar ausgebildet sind.

11. Verfahren zur Durchführung eines Integritätstests an einer Probe von Einwegkomponenten mit den Schritten:
- Bereitstellen einer Mediumkammer (10) mit einem nach außen begrenzten Innenraum (11);
- Anordnen der Probe (100) in einer Probenhalterung (20) derart, dass die Probe (100) die Mediumkammer (10) zumindest teilweise nach außen begrenzt;
- Ausrichten der Mediumkammer (10) in einer Befüllposition derart, dass eine Entlüftungsleitung (19) der Mediumkammer (10) im Wesentlichen am höchsten Punkt des Innenraums (11) der Mediumkammer (10) angeordnet ist, wobei die Probenhalterung (20) vertikal unter der Entlüftungsleitung (19) angeordnet ist;
- Befüllen des Innenraums (11) der Mediumkammer (10) mit einem Kulturmedium bei geöffneter Entlüftungsleitung (19) mittels einer Mediumzuleitung (15) der Mediumkammer (10); und
- Ausrichten der Mediumkammer (10) in einer Suspensionsbefüllposition und/oder in einer Beaufschlagungsposition, in der die Probenhalterung (20) vertikal oberhalb der Entlüftungsleitung (19) angeordnet ist.

12. Verfahren nach Anspruch 11, mit den Schritten:
- Ausrichten der Mediumkammer (10) in einer Beaufschlagungsposition, in der die Probe mit einer zur Horizontalen geneigten Probenaußenfläche ausgerichtet ist und
- Beaufschlagen der Probe in der Beaufschlagungsposition von außen mit Bakterien und/oder Sporen.

13. Verfahren nach Anspruch 12, wobei die Probe in der Beaufschlagungsposition unmittelbar mit einem Bakterien- und/oder Sporenaerosol beaufschlagt wird.

14. Verfahren nach einem der Ansprüche 11 bis 13, mit den Schritten:
- Bereitstellen einer Suspensionskammer (50) derart, dass ein Suspensionskammerinnenraum (51) vom Innenraum (11) der Mediumkammer (10) zumindest teilweise nur durch die in der Probenhalterung (20) angeordnete Probe (100) getrennt ist;
- Ausrichten der Vorrichtung (1; 2; 3; 4; 5) in der Suspensionsbefüllposition derart, dass eine Suspensionskammerentlüftungsleitung (59) im Wesentlichen am höchsten Punkt des Suspensionskammerinnenraums (51) angeordnet ist,
- Befüllen des Suspensionskammerinnenraums (51) in der Suspensionsbefüllposition mit einer Bakterien- und/oder Sporensuspension bei geöffneter Suspensionskammerentlüftungsleitung (59).

15. Verwendung einer Vorrichtung (1; 2; 3; 4; 5) nach einem der Ansprüche 1 bis 10 zum Durchführen eines Integritätstests an einer Probe (100) von Einwegkomponenten gemäß einem Verfahren nach einem der Ansprüche 11 bis 14.

## Claims

1. A device for carrying out an integrity test on a sample of single-use components, comprising
- a medium chamber (10) having an outwardly delimited inner space (11) for receiving a culture medium;
- a sample holder (20) in which the sample (100) can be arranged such that the sample (100) delimits the medium chamber (10) at least partially outwards; and
- an alignment device (40);
wherein
- the medium chamber (10) has a vent line (19), which is arranged in a sealable and openable manner on one side of the medium chamber (10) facing away from the sample holder (20);
- the medium chamber (10) can be aligned by means of the alignment device (40) in a filling position, in which
- the vent line (19) of the medium chamber (10) is substantially arranged at the highest point of the inner space (11) of the medium chamber (10),
- the medium chamber (10) can be filled with the culture medium by means of a medium supply line (15) of the medium chamber (10), and
- in which filling position the sample holder (20) is arranged vertically below the vent line (19); and
- the medium chamber (10) can, in addition, be aligned by means of the alignment device (40) in a suspension filling position and/or in an application position, in which the sample holder (20) is arranged vertically above the vent line (19).

2. The device according to Claim 1, wherein the medium chamber (10) can be aligned by means of the alignment device (40):
- in the suspension filling position such that the sample holder (20) is arranged vertically above the vent line (19), and
- in addition, in an application position such that the sample is arranged with its sample outer face inclined to the horizontal.

3. The device according to Claim 1 or 2, wherein the vent line (19) is arranged in the filling position of the medium chamber (10) at the vertically highest point of the medium chamber (10) and the sample holder (20) is arranged at the vertically lowest point of the medium chamber (10).

4. The device according to any one of the preceding claims, having a connecting element for connecting the medium chamber (10) to a suspension chamber (50) in such a manner that the sample (100) arranged in the sample holder (20) provides an interface between the inner space of the medium chamber (10) and a suspension chamber inner space (51) of the suspension chamber (50).

5. The device according to Claim 4, having a suspension chamber vent line (59) of the suspension chamber (50) which is arranged on one side of the suspension chamber (50) facing away from the sample holder (20).

6. The device according to any one of the preceding claims, wherein at least one transparent region is configured in a side wall (12) of the medium chamber (10).

7. The device according to any one of the preceding claims, wherein the sample holder (20) is arranged in a detachable connection with the medium chamber (10).

8. The device according to any one of the preceding claims, wherein the device (1; 2; 3; 4; 5) is configured in a pressure-stable manner from a negative pressure of approx. 1*10^{∼10} mbar up to an excess pressure of approx. 2 bar.

9. The device according to any one of the preceding claims, having at least one electrode for contacting the inner space (11) of the medium chamber (10).

10. The device according to any one of the preceding claims, wherein supply and discharge lines (15, 16, 17, 19, 55, 56, 57, 59) of the device (1; 2; 3; 4; 5) are configured in a sealable manner.

11. A method for carrying out an integrity test on a sample of single-use components having the steps of:
- providing a medium chamber (10) with an outwardly delimited inner space (11);
- arranging the sample (100) in a sample holder (20) in such a manner that the sample (100) at least partially delimits the medium chamber (10) outwardly;
- aligning the medium chamber (10) in a filling position in such a manner that a vent line (19) of the medium chamber (10) is substantially arranged at the highest point of the inner space (11) of the medium chamber (10), wherein the sample holder (20) is arranged vertically below the vent line (19);
- filling the inner space (11) of the medium chamber (10) with a culture medium when the vent line (19) is opened by means of a medium supply line (15) of the medium chamber (10); and
- aligning the medium chamber (10) in a suspension filling position and/or in an application position, in which the sample holder (20) is arranged vertically above the vent line (19).

12. The method according to Claim 11, having the steps of:
- aligning the medium chamber (10) in an application position, in which the sample is aligned with a sample outer face inclined to the horizontal, and
- acting on the sample in the application position from outside with bacteria and/or spores.

13. The method according to Claim 12, wherein the sample is acted upon in the application position directly with a bacteria and/or spore aerosol.

14. The method according to any one of Claims 11 to 13, having the steps of:
- providing a suspension chamber (50) in such a manner that a suspension chamber inner space (51) is only separated from the inner space (11) of the medium chamber (10) at least partially by the sample (100) arranged in the sample holder (20);
- aligning the device (1; 2; 3; 4; 5) in the suspension filling position in such a manner that a suspension chamber vent line (59) is substantially arranged at the highest point of the suspension chamber inner space (51),
- filling the suspension chamber inner space (51) in the suspension filling position with a bacteria and/or spore suspension when the suspension chamber vent line (59) is opened.

15. Use of a device (1; 2; 3; 4; 5) according to any one of Claims 1 to 10 for carrying out an integrity test on a sample (100) of single-use components in accordance with a method according to any one of Claims 11 to 14.

## Revendications

1. Dispositif pour l'exécution d'un test d'intégrité sur un échantillon de composants à usage unique, comprenant :
- une chambre à fluide (10) avec un espace intérieur (11) délimité vers l'extérieur pour la réception d'un milieu de culture ;
- un support d'échantillon (20) dans lequel l'échantillon (100) peut être disposé de manière à ce que l'échantillon (100) délimite au moins partiellement la chambre à fluide (10) vers l'extérieur ; et
- un dispositif d'orientation (40) ;
dans lequel
- la chambre à fluide (10) présente une conduite d'aération (19) disposée de manière à pouvoir être fermée et ouverte sur un côté de la chambre à fluide (10) opposé au support d'échantillon (20) ;
- la chambre à fluide (10) peut être orientée dans une position de remplissage à l'aide du dispositif d'orientation (40), dans laquelle
- la conduite d'aération (19) de la chambre à fluide (10) est disposée quasiment au plus haut point de l'espace intérieur (11) de la chambre à fluide (10),
- la chambre à fluide (10) peut être remplie avec le milieu de culture par le biais d'une conduite d'alimentation en fluide (15) de la chambre à fluide (10), et
- dans laquelle le support d'échantillon (20) est disposé verticalement sous la conduite d'aération (19) ; et
- la chambre à fluide (10) peut en outre être orientée à l'aide du dispositif d'orientation (40) dans une position de remplissage de suspension et/ou dans une position de sollicitation, dans laquelle le support d'échantillon (20) est disposé verticalement au-dessus de la conduite d'aération (19).

2. Dispositif selon la revendication 1, dans lequel la chambre à fluide (10) peut, grâce au dispositif d'orientation (40) :
- être orientée dans la position de remplissage de suspension de manière à ce que le support d'échantillon (20) soit disposé verticalement au-dessus de la conduite d'aération (19), et
- être en outre orientée dans la position de sollicitation de manière à ce que l'échantillon soit disposé avec sa surface extérieure inclinée par rapport à l'horizontale.

3. Dispositif selon la revendication 1 ou 2, dans lequel la conduite d'aération (19) est disposée verticalement au plus haut point de la chambre à fluide (10) dans la position de remplissage de la chambre à fluide (10), et le support d'échantillon (20) est disposé au point le plus bas de la chambre à fluide (10).

4. Dispositif selon l'une des revendications précédentes, comprenant un élément de liaison destiné à relier la chambre à fluide (10) à une chambre à suspension (50), de telle façon que l'échantillon (100) disposé dans le support d'échantillon (20) présente une surface de délimitation entre l'espace intérieur de la chambre à fluide (10) et un espace intérieur de chambre à suspension (51) de la chambre à suspension (50).

5. Dispositif selon la revendication 4, comprenant une conduite d'aération de chambre à suspension (59) de la chambre à suspension (50), laquelle est disposée sur un côté de la chambre à suspension (50) opposé au support d'échantillon (20).

6. Dispositif selon l'une des revendications précédentes, dans lequel au moins une région transparente est formée dans une paroi latérale (12) de la chambre à fluide (10).

7. Dispositif selon l'une des revendications précédentes, dans lequel le support d'échantillon (20) est disposé dans une liaison amovible avec la chambre à fluide (10).

8. Dispositif selon l'une des revendications précédentes, dans lequel le dispositif (1 ; 2 ; 3 ; 4 ; 5) est conçu de manière à être stable à la pression, d'une sous-pression d'environ 1*10⁻¹⁰ mbar jusqu'à une surpression d'environ 2 bar.

9. Dispositif selon l'une des revendications précédentes, comprenant au moins une électrode pour la mise en contact de l'espace intérieur (11) de la chambre à fluide (10).

10. Dispositif selon l'une des revendications précédentes, dans lequel des conduites d'arrivée et d'évacuation (15, 16, 17, 19, 55, 56, 57, 59) du dispositif (1 ; 2 ; 3 ; 4 ; 5) sont conçues de manière à pouvoir être fermées.

11. Procédé pour l'exécution d'un test d'intégrité sur un échantillon de composants à usage unique, comprenant les étapes suivantes :
- mise à disposition d'une chambre à fluide (10) avec un espace intérieur (11) délimité vers l'extérieur ;
- disposition de l'échantillon (100) dans un support d'échantillon (20) de manière à ce que l'échantillon (100) délimite au moins partiellement la chambre à fluide (10) vers l'extérieur ;
- orientation de la chambre à fluide (10) dans une position de remplissage, de manière à ce que la conduite d'aération (19) de la chambre à fluide (10) soit disposée quasiment au plus haut point de l'espace intérieur (11) de la chambre à fluide (10), le support d'échantillon (20) étant disposé verticalement sous la conduite d'aération (19);
- remplissage de l'espace intérieur (11) de la chambre à fluide (10) avec un milieu de culture lorsque la conduite d'aération (19) est ouverte, à l'aide d'une conduite d'alimentation en fluide (15) de la chambre à fluide (10); et
- orientation de la chambre à fluide (10) dans une position de remplissage de suspension et/ou dans une position de sollicitation, dans laquelle le support d'échantillon (20) est disposé verticalement au-dessus de la conduite d'aération (19).

12. Procédé selon la revendication 11, comprenant les étapes suivantes :
- orientation de la chambre à fluide (10) dans une position de sollicitation dans laquelle l'échantillon est orienté avec une surface extérieure d'échantillon inclinée par rapport à l'horizontale, et
- sollicitation de l'échantillon dans la position de sollicitation depuis l'extérieur avec des bactéries et/ou des spores.

13. Procédé selon la revendication 12, dans lequel l'échantillon dans la position de sollicitation est sollicité directement avec un aérosol à bactéries et/ou à spores.

14. Procédé selon l'une des revendications 11 à 13, comprenant les étapes suivantes :
- mise à disposition d'une chambre à suspension (50) de telle façon qu'un espace intérieur de chambre à suspension (51) est séparé au moins partiellement de l'espace intérieur (11) de la chambre à fluide (10) uniquement par l'échantillon (100) disposé dans le support d'échantillon (20) ;
- orientation du dispositif (1 ; 2 ; 3 ; 4 ; 5) dans la position de remplissage de suspension de telle façon qu'une conduite d'aération de chambre à suspension (59) est disposée quasiment au plus haut point de l'espace intérieur de chambre à suspension (51),
- remplissage de l'espace intérieur de chambre à suspension (51) dans la position de remplissage de suspension avec une suspension de bactéries et/ou de spores lorsque la conduite d'aération de chambre à suspension (59) est ouverte.

15. Utilisation d'un dispositif (1 ; 2 ; 3 ; 4 ; 5) selon l'une des revendications 1 à 10 pour l'exécution d'un test d'intégrité sur un échantillon (100) de composants à usage unique à l'aide d'un procédé selon l'une des revendications 11 à 14.
